# EUROPEAN PATENT APPLICATION

(11) **EP 4 772 211 A1**
(43) Date of publication of application: **08.07.2026**
(21) Application number: 26150250.4
(22) Date of filing: 05.01.2026
(51) Int. Cl.: A61M 5/142, A61M 39/10

(54) **DOWNSTREAM PRESSURE RELIEF**

(30) Priority: 06.01.2025 US 202563742078 P
(71) Applicant: EITAN MEDICAL LTD., 4250529 Netanya (IL)
(72) Inventor: GOFMAN, Evgeny, Givataim, 5332712 (IL); EITAN, Boaz, Hofit, 4029500 (IL); MEIDAN, Elad, Binyamina Givat Ada, 3780800 (IL)
(74) Representative: Evens, Paul Jonathan

(57) **Abstract**

Infusion tubing (20) has an upstream segment (26) upstream of a pump (24) and a downstream segment (28) downstream of the pump. A side-tube (22) filled with air (36) has a proximal end (30) in fluid communication with the downstream segment and a sealed distal end (32). When pressure in the downstream segment increases, therapeutic substance (21) enters the side-tube, thereby compressing the air and reducing the pressure within the downstream segment. When the pressure in the downstream segment decreases, the compressed air pushes the therapeutic substance back into the downstream segment. An inner diameter (D1) of the side-tube is sufficiently small such that in the absence of positive pressure within the downstream segment, surface tension at the proximal end of the side-tube prevents the therapeutic substance from flowing into the side-tube regardless of the orientation of the side-tube with respect to gravity. Other applications are also described.

## Description

### CROSS REFERENCE TO RELATED APPLICATION

The present application claims priority to US 63/742,078 to Gofman et al., filed January 6, 2025, entitled "Downstream pressure relief," and which is incorporated herein by reference.

### FIELD OF THE INVENTION

The present invention relates generally to medical fluiddelivery devices, and more specifically to infusion pumps.

### BACKGROUND

Pumps are often used in the medical industry for delivering fluids, e.g., drugs, or diagnostic fluids to a subject. One type of medical pump is an infusion pump, used to infuse a fluid into a subject's circulatory system via infusion tubing. Some infusion pumps pump the fluid in a pulsatile manner through the infusion tubing and may include a mechanism for damping the pulsations, i.e., absorbing pressure spikes within the pulsatile flow.

US Patent Application Publication 2005/0089428 to Navarro describes a pump delivery system including a pump mechanism for delivering liquids under pressure while also producing a pulsating output during operation, and a pump pulsation dampening attachment for dampening the pulsating output of the pump mechanism. The pump pulsation dampening assembly includes a pressurized vessel having an interior surface defining a chamber for receiving the liquid from the pump mechanism, the chamber also containing a pocket of pressurized air therein for absorbing the pulsating output generated by the pump mechanism. Other embodiments are also described.

US Patent Application Publication 2011/0005630 to Al-Khomairi describes systems and apparatus for suppressing/controlling pressure spikes in a fluid pipe system. In one aspect, an apparatus for controlling pressure spikes in a fluid pipe system includes, for example, a fluid pressure spike suppression pipe ("damper pipe") portion with multiple openings for connecting to at least two network pipes in a fluid system pipe network. The damper pipe has a diameter that is larger than respective diameters of the network pipes within which fluid pressure spikes are to be suppressed. First and second openings for connecting to the network pipes are respectively positioned at proximal and distal ends of the damper pipe. The first opening in the damper pipe is for fluid ingress into the damper pipe via a first pipe network pipe. The second opening in the damper pipe is for fluid egress out of the damper pipe and into a second network pipe. Other embodiments are also described.

US Patent Application Publication 2011/0196303 to Chan et al., describes a medical fluid infusion system including: a fluid pathway for transporting a pulsatile flow of fluid; a dampening element in communication with the fluid pathway, the dampening element configured to actively dampen pressure fluctuations of the pulsatile flow to smoothen the pulsatile fluid flow, the dampening element described as operable in any orientation; and a fluid flow sensor disposed along the fluid pathway downstream of the dampening element to measure the flow rate of the smoothened fluid flow. Other embodiments are also described.

US Patent Application Publication 2020/0200313 to Chu et al., describes a fluid management system that may include a fluid pump capable of generating a pulsatile flow of fluid, a fluid pathway for transporting the pulsatile flow of fluid from a fluid source through the fluid pump to a medical device, a dampening element in fluid communication with the fluid pathway and operably independent of the fluid pump, the dampening element comprising one or more barrels, each barrel including a movable seal member disposed within the barrel and a biasing member disposed within the barrel and engaged with the movable seal member, the dampening element being responsive to pressure fluctuations of the pulsatile fluid flow to actively dampen the pressure fluctuations, and a fluid flow sensor disposed along the fluid pathway between the dampening element and the medical device to measure a flow rate of the smoothened pulsatile fluid flow in both flow directions. Other embodiments are also described.

US Patent Application Publication 2022/0273870 to Feith describes an apparatus for coupling to a fluid delivery system and for receiving a fluid into the apparatus; a portion of the apparatus permitting a change in shape or size to accommodate the fluid volume or pressure received therein. The apparatus includes a housing with a cavity, an expandable reservoir with an opening and a passage, and a cap; the expandable reservoir positioned within the cavity and coupled to a fluid source. The expandable reservoir includes an unrestrained orientation when a fluid volume or pressure therein is below a threshold, and an expanded orientation, when the fluid volume or pressure therein is above the threshold; the expandable reservoir moving toward the expanded orientation to accommodate a fluid volume or pressure received through an opening, and the expandable reservoir moving toward the unrestrained orientation to direct a fluid volume or pressure through an opening. Other embodiments are also described.

US Patent Application Publication 2023/0390485 to Ovadia et al., describes methods and systems for use with peristaltic pumps. More particularly, described in the application are peristaltic pump systems that include a dampener for reducing the pulsations of the flowrate from the peristaltic pump system to produce, mix, transfer and/or manufacture pharmaceutical compositions and formulations, including pharmaceutical compositions and formulations comprising lipids and RNA. Other embodiments are also described.

US Patent 5639219 to Conatser describes an airless paint sprayer includes an inlet check valve assembly having a dual spring configuration which allows for increased travel distance of the inlet check valve thereby increasing the fluid flow through the valve without diminishing response time of the valve or the priming operations of the system. A dampener also contributes to lessening the cavitation and loss of pressure in the paint sprayer and comprises a generally T-shaped fitting in which one leg of the T-shaped fitting is capped and includes a trapped volume of air to dampen acceleration/deceleration forces and acceleration spikes being transmitted from the pump to the fluid being pumped through the fitting. Other embodiments are also described.

### SUMMARY OF THE INVENTION

In accordance with some applications of the present invention, infusion tubing is provided for use with a pump that operates in a pulsatile manner to pump a therapeutic substance to a subject. Due to the pulsatile flow of the therapeutic substance, pressure peaks may occur within the infusion tubing downstream of the pump. In order to damp these pressure spikes a side-tube is provided that branches off the infusion tubing downstream of the pump. The side-tube is sealed at its distal end and is filled with air. When a pressure spike occurs in the infusion tubing, some of the therapeutic substance enters the side-tube and compresses the air within the side-tube. This diversion of some of the therapeutic substance to the side-tube reduces the pressure within the infusion tubing. When the fluid pressure decreases, the compressed air then pushes the diverted therapeutic substance back into the downstream flow of the therapeutic substance.

Using a side-tube filled with air without including a barrier (e.g., a stretchable septum) separating the air from the therapeutic substance at the entrance of the side-tube could cause therapeutic substance to unintentionally, e.g., when no pressure peak is present in the downstream flow, enter the side-tube due to gravity if the side-tube is not maintained above the infusion tubing with respect to the direction of gravity. In order to avoid this, the side-tube of some applications of the present invention has an inner diameter that is sufficiently small, e.g., less than 3.1 mm, such that surface tension of the therapeutic substance at the entrance to the side tube prevents the therapeutic substance from flowing into the side-tube unless there is a positive pressure pushing the therapeutic substance into the side-tube. Thus, the surface tension of the therapeutic substance acts a "barrier" preventing the therapeutic substance from flowing into the side-tube (unless under positive pressure) regardless of the orientation of the side-tube with respect to gravity.

Furthermore, using a side-tube filled with air without including a barrier separating the air from the therapeutic substance might cause unintentional mixing of the air into the therapeutic substance over time. Thus, for some applications, the diameter of the side tube is also sufficiently small such that the surface tension of the therapeutic substance inhibits air within the side-tube mixing with the therapeutic substance, even while the therapeutic substance is inside the side-tube due to a pressure peak in the downstream flow.

There is therefore provided, in accordance with some applications of the present invention apparatus for use with a pump configured to operate in a pulsatile manner to pump a therapeutic substance to a subject, the apparatus including:
infusion tubing configured to be coupled to the pump for transporting a pulsatile flow of the therapeutic substance to the subject, the infusion tubing having an upstream segment upstream of the pump and a downstream segment downstream of the pump; and
a side-tube which (i) has a proximal end that is in fluid communication with the downstream segment of the infusion tubing, (ii) has a distal end that is sealed, and (iii) is filled with air, wherein:
   (a) when fluid pressure in the downstream segment of the infusion tubing increases, some of the therapeutic substance in the downstream segment of the infusion tubing enters the side-tube via the proximal end of the side-tube, thereby compressing the air within the side-tube and reducing the pressure within the downstream segment of the infusion tubing, and
   (b) subsequently, when the fluid pressure in the downstream segment of the infusion tubing decreases, the compressed air pushes the therapeutic substance disposed within the side-tube back into the downstream segment of the infusion tubing,
      wherein an inner diameter of the side-tube is sufficiently small such that in the absence of positive pressure within the downstream segment of the infusion tubing, surface tension of the therapeutic substance at the proximal end of the side-tube prevents the therapeutic substance from flowing into the side-tube regardless of the orientation of the side-tube with respect to gravity.

For some applications, the inner diameter of the side-tube is sufficiently small such that the surface tension of the therapeutic substance inhibits air within the side-tube mixing with the therapeutic substance under positive pressure when therapeutic substance is disposed within the side-tube.

For some applications, the inner diameter of the side-tube is less than 3.1 mm.

For some applications, a length of the side tube is 5-50 cm.

For some applications, a length of the side tube is 50 - 200 cm.

For some applications, the inner diameter of the side-tube is less than an inner diameter of the infusion tubing.

For some applications, the inner diameter of the side-tube is greater than or equal to an inner diameter of the infusion tubing.

There is further provided, in accordance with some applications of the present invention, apparatus for use with a pump configured to operate in a pulsatile manner to pump a therapeutic substance to a subject, the apparatus including:
infusion tubing configured to be coupled to the pump for transporting a pulsatile flow of the therapeutic substance to the subject, the infusion tubing having an upstream segment upstream of the pump and a downstream segment downstream of the pump; and
a side-tube which (i) has a proximal end that is in fluid communication with the downstream segment of the infusion tubing, (ii) has a distal end that is sealed, and (iii) is filled with air, wherein:
   (a) when fluid pressure in the downstream segment of the infusion tubing increases, some of the therapeutic substance in the downstream segment of the infusion tubing enters the side-tube via the proximal end of the side-tube, thereby compressing the air within the side-tube and reducing the pressure within the downstream segment of the infusion tubing, and
   (b) subsequently, when the fluid pressure in the downstream segment of the infusion fluid decreases, the compressed air pushes the therapeutic substance disposed within the side-tube back into the downstream segment of the infusion tubing,
      wherein an inner diameter of the side-tube is less than 3.1 mm.

For some applications, a length of the side tube is 5-50 cm.

For some applications, a length of the side tube is 50 - 200 cm.

For some applications, the inner diameter of the side-tube is sufficiently small such that surface tension of the therapeutic substance inhibits air within the side-tube mixing with the therapeutic substance under positive pressure when therapeutic substance is disposed within the side-tube.

For some applications, the inner diameter of the side-tube is less than an inner diameter of the infusion tubing.

For some applications, the inner diameter of the side-tube is greater than or equal to an inner diameter of the infusion tubing.

The present invention will be more fully understood from the following detailed description of applications thereof, taken together with the drawings, in which:

### BRIEF DESCRIPTION OF THE DRAWINGS

Figs. 1A-B are schematic illustrations of infusion tubing and a side-tube, in accordance with some applications of the present invention;
Figs. 2A-C are schematic illustrations showing the side-tube in accordance with some applications of the present invention;
Fig. 3 is a graph illustrating experimental data, in accordance with some applications of the present invention; and
Fig. 4 shows the side-tube rolled up inside a housing, in accordance with some applications of the present invention.

### DETAILED DESCRIPTION

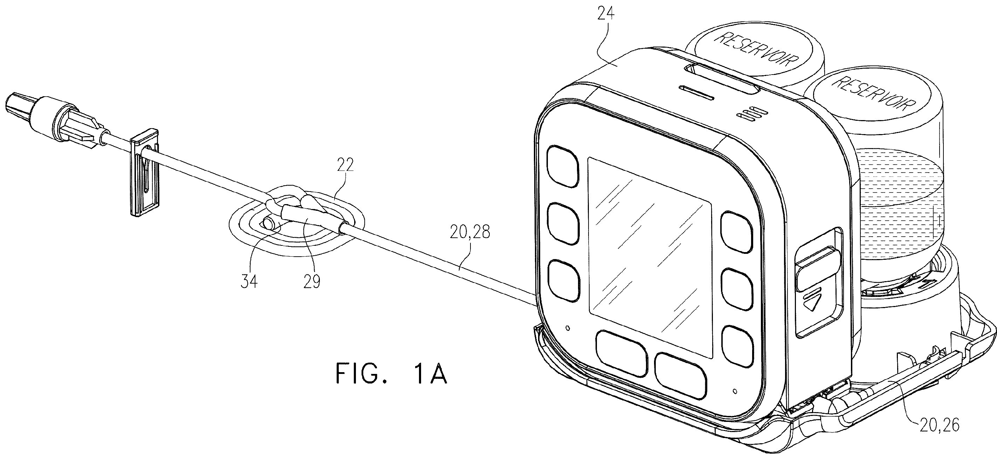

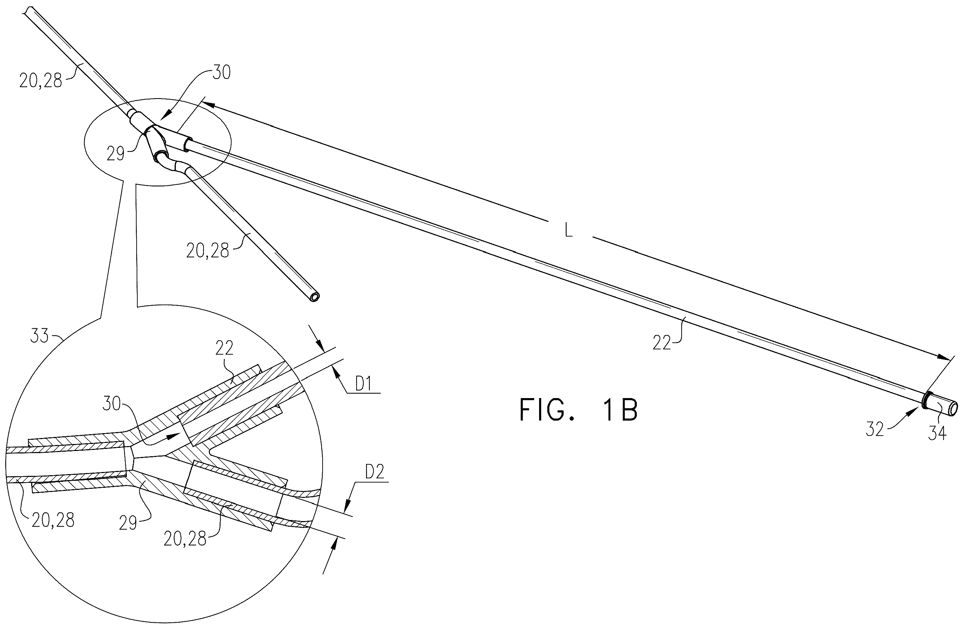

Reference is now made to Figs. 1A-B, which are schematic illustrations of infusion tubing 20 and a side-tube 22, in accordance with some applications of the present invention. Infusion tubing 20 is configured to be coupled to a pump 24, e.g., an infusion pump, for transporting a pulsatile flow of a therapeutic substance 21 to a subject. An upstream segment 26 of infusion tubing 20 is upstream of pump 24 and a downstream segment 28 of infusion tubing 20 is downstream of pump 24 (as shown in Fig. 1A). Side-tube 22 has a proximal end 30 that is in fluid communication with downstream segment 28 of infusion tubing 20. For some applications, proximal end 30 of side-tube 22 is in fluid communication with downstream segment 28 via a tube-connector 29, e.g., a Y-connector, as shown in enlarged view circle 33 in Fig. 1B, or a T-connector. For some applications, downstream segment 28 of infusion tubing 20 is itself split into two downstream tube segments 28 that are in fluid communication with each other also via tube-connector 29. Alternatively, downstream segment 28 of infusion tubing 20 may be implemented as one long tube with side-tube 22 attached to an opening in the wall of downstream segment 28 and sealed around the opening (not shown). Alternatively, downstream segment 28 of infusion tubing 20 and side-tube 22 may be made of a unitary piece of tubing shaped to define downstream segment 28 and side-tube 22 (not shown). A distal end 32 of infusion tubing 20 is sealed, e.g., with a plug 34.

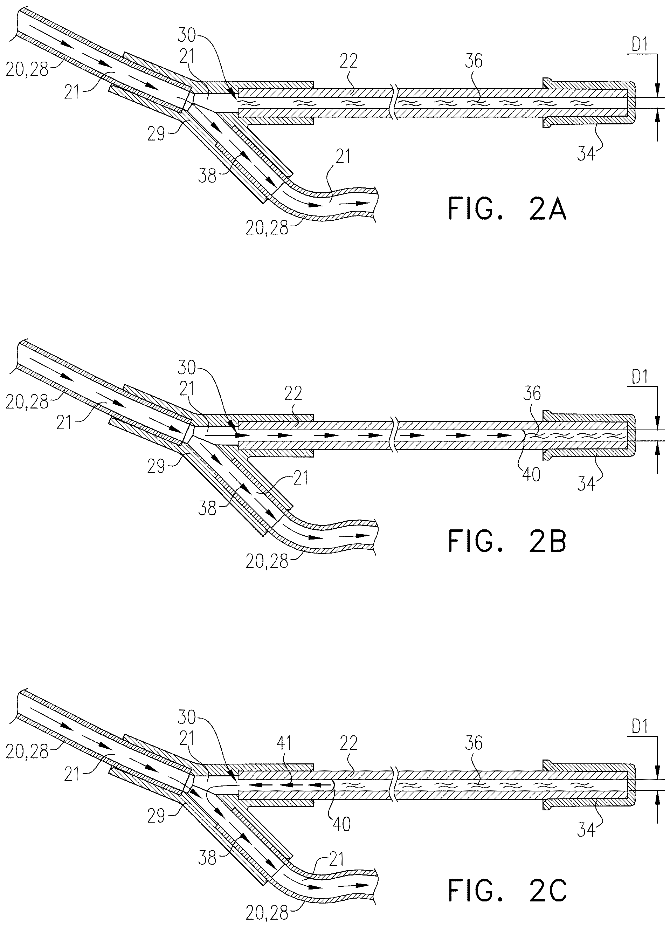

Reference is now made to Figs. 2A-C, which are schematic illustrations showing side-tube 22 in accordance with some applications of the present invention. As described hereinabove, side-tube 22 acts as a damper for pressure peaks within the pulsatile flow of therapeutic substance 21 downstream of pump 24. Side-tube 22 is filled with air 36. In the absence of positive pressure, e.g., from a pressure peak within the pulsatile flow, therapeutic substance 21 flows downstream through downstream segment 28 of infusion tubing 20, as illustrated by arrows 38 in Fig. 2A indicating the flow therapeutic substance 21. When fluid pressure in downstream segment 28 of infusion tubing 20 increases, some of therapeutic substance 21 in downstream segment 28 of infusion tubing 20 flows into side-tube 22 via proximal end 30 of side-tube 22 (as illustrated by arrows 39 in Fig. 2B), thereby compressing air 36 within side-tube 22 and reducing the pressure within downstream segment 28 of infusion tubing 20. Subsequently, when the fluid pressure in downstream segment 28 of infusion tubing 20 decreases, compressed air 36 pushes therapeutic substance 21 disposed within the side-tube back into downstream segment 28 of infusion tubing 20 (as illustrated by arrows 41 in Fig. 2C).

As described hereinabove, the inventors have realized that a solution to inhibiting therapeutic substance 21 from unintentionally flowing into side-tube 22, e.g., in the absence of a positive pressure peak in the pulsatile flow of therapeutic substance 21, an inner diameter D1 of side-tube 22 is sufficiently small, e.g., less than 3.1 mm, such that in the absence of positive pressure within downstream segment 28 of infusion tubing 20, surface tension of therapeutic substance 21 at proximal end 30 of side-tube 22 prevents therapeutic substance 21 from flowing into side-tube 22 regardless of the orientation of the side-tube with respect to gravity. This means, for example, that if side-tube 22 is oriented vertically with respect to gravity such that proximal end 30 is at the top, air 36 will not float upwards, i.e., will not exit side-tube 22 to be displaced by therapeutic substance 21, due to the surface tension of therapeutic substance 21 at proximal end 30 of side-tube 22. For some applications, inner diameter D1 of side-tube 22 is sufficiently small such that even when therapeutic substance 21 is pushed into side-tube 22, i.e., under positive pressure, the surface tension of therapeutic substance 21 (illustrated by line 40 in Figs. 2B-C) acts as a barrier between therapeutic substance 21 and air 36, and thus inhibits air 36 within side-tube 22 mixing with therapeutic substance 21.

For some applications, such as is illustrated in Fig. 1B, inner diameter D1 is smaller than an inner diameter D2 of infusion tubing 20. However, it is noted that the above-described advantages relating to inner diameter D1 of side-tube 22 being sufficiently small are based on the absolute size of inner diameter D1, and are independent of the inner diameter D2 of infusion tubing 20. Thus, for some applications, inner diameter D1 of side-tube 22 may also be equal to or larger than inner diameter D2 of infusion tubing 20.

It is noted that since inner diameter D1 is small, in order for there to be room for a sufficient volume of therapeutic substance 21 to flow into side-tube 22 in order to reduce the pressure in downstream segment 28 of infusion tubing 20, a length L of side-tube 22 is relatively long. For example, for some applications, length L of side-tube 22 is at least 5 cm and/or less than 50 cm. For some applications, length L of side-tube 22 is at least 50 cm and/or less than 200 cm. Fig. 1B shows side-tube 22 in a straight configuration in order to illustrate length L relative to inner diameter D1 of side-tube 22. It is noted that while Fig. 2B shows length L to be considerably larger than inner diameter D1 for illustrative purposes, Fig. 1B is not necessarily drawn to scale.

The inventors have realized that based on different flow conditions, e.g., viscosity of therapeutic substance 21, flow rate, and pressure requirements, different combinations of parameters pertaining to side-tube 22 may be used, e.g., different combinations of length L, inner diameter D1, the material of side-tube 22, and/or outer diameter of side-tube 22. For example, the tube thickness and material should be such that the tube expansion under pressure is small enough that inner diameter D1 does not change under high pressure preconditions in a manner that would allow flow of therapeutic substance 21 past air 36. For some applications, side-tube 22 may be made of silicone or a polymer material, e.g., polyvinyl chloride (PVC) or thermoplastic elastomer (TPE). Additionally, the pressure-reduction capability within side-tube 22 depends on the volume of air 36 within side-tube 22, which depends on the combination of inner diameter D1 and length L.

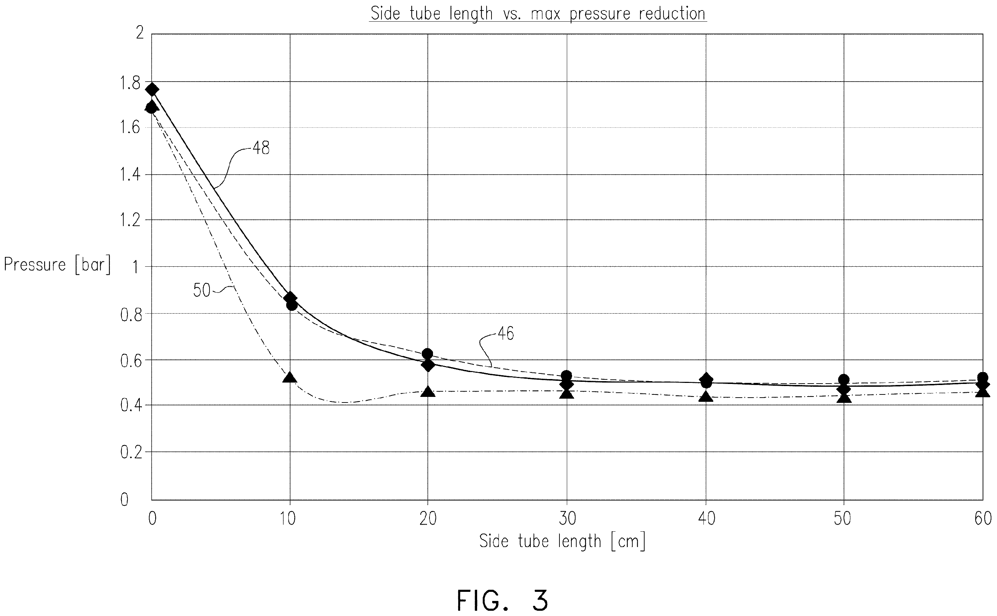

Reference is now made to Fig. 3, which is a graph showing experimental data collected by the inventors relating to the pressure reduction in downstream segment 28 of infusion tubing 20 for different combinations of length L, inner diameter D1, and an outer diameter of side-tube 22 under a given set of flow conditions. For the specific experiment illustrated by Fig. 3, the flow conditions were a flow rate of 240 mL/h through four parallel 24G catheters, a fluid viscosity of 17 cP, and a fluid temperature of 23 degrees Celsius. The experiment was repeated three times using (i) a tube having an inner diameter D1 of 1.5 mm and an outer diameter of 3 mm, represented by solid line 46 with circular data points, (ii) a tube having an inner diameter D1 of 1.5 mm and an outer diameter of 2 mm, represented by dashed line 48 with diamond-shaped data points, and (iii) a tube having an inner diameter D1 of 2 mm and an outer diameter of 2.5 mm, represented by dash-dot line 50 with triangular data points. All of the tubes used in the experiment were made of PVC. As illustrated by the experimental data, for all three of the tubes that were tested, a significant reduction in pressure is already achieved with a length L of 5 cm. The pressure reduction increased as the tube length L increased until a length L of 30 cm for the tubes having inner diameter D1 of 1.5 mm and until a length L of around 10 cm for the tube having inner diameter D1 of 2.5 mm. The pressure reduction remained substantially constant with lengths L longer than 30 cm.

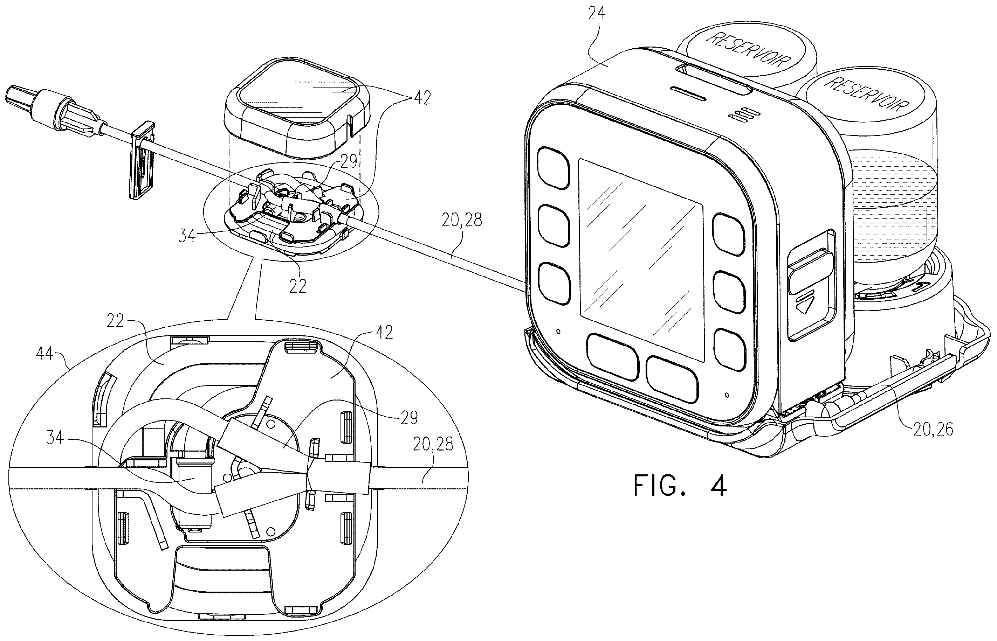

Reference is now made to Fig. 4, which shows side-tube 22 rolled up inside a housing 42, in accordance with some applications of the present invention. Since length L of side-tube 22 is relatively long (as described hereinabove), for some applications, in order to save space, side-tube 22 may be rolled up inside housing 42 which is coupled to infusion tubing 20. Enlarged view circle 44 in Fig. 4 shows housing 42 without its top cover in order to illustrate how side-tube 22 is rolled up inside the housing along with tube-connector 29 and plug 34.

It will be appreciated by persons skilled in the art that the present invention is not limited to what has been particularly shown and described hereinabove. Rather, the scope of the present invention includes both combinations and subcombinations of the various features described hereinabove, as well as variations and modifications thereof that are not in the prior art, which would occur to persons skilled in the art upon reading the foregoing description.

## Claims

1. Apparatus for use with a pump configured to operate in a pulsatile manner to pump a therapeutic substance to a subject, the apparatus comprising:
infusion tubing configured to be coupled to the pump for transporting a pulsatile flow of the therapeutic substance to the subject, the infusion tubing having an upstream segment upstream of the pump and a downstream segment downstream of the pump; and
a side-tube which (i) has a proximal end that is in fluid communication with the downstream segment of the infusion tubing, (ii) has a distal end that is sealed, and (iii) is filled with air, wherein:
(a) when fluid pressure in the downstream segment of the infusion tubing increases, some of the therapeutic substance in the downstream segment of the infusion tubing enters the side-tube via the proximal end of the side-tube, thereby compressing the air within the side-tube and reducing the pressure within the downstream segment of the infusion tubing, and
(b) subsequently, when the fluid pressure in the downstream segment of the infusion tubing decreases, the compressed air pushes the therapeutic substance disposed within the side-tube back into the downstream segment of the infusion tubing,
wherein an inner diameter of the side-tube is (a) sufficiently small such that in the absence of positive pressure within the downstream segment of the infusion tubing, surface tension of the therapeutic substance at the proximal end of the side-tube prevents the therapeutic substance from flowing into the side-tube regardless of the orientation of the side-tube with respect to gravity, and/or (b) less than 3.1 mm.

2. The apparatus according to claim 1, wherein the inner diameter of the side-tube is sufficiently small such that the surface tension of the therapeutic substance inhibits air within the side-tube mixing with the therapeutic substance under positive pressure when therapeutic substance is disposed within the side-tube.

3. The apparatus according to any one of claims 1-2, wherein the inner diameter of the side-tube is sufficiently small such that in the absence of positive pressure within the downstream segment of the infusion tubing, surface tension of the therapeutic substance at the proximal end of the side-tube prevents the therapeutic substance from flowing into the side-tube regardless of the orientation of the side-tube with respect to gravity.

4. The apparatus according to claim 3, wherein the inner diameter of the side-tube is less than 3.1 mm.

5. The apparatus according to any one of claims 1-2, wherein the inner diameter of the side-tube is less than 3.1 mm.

6. The apparatus according to any one of claims 1-5, wherein a length of the side tube is 5-50 cm.

7. The apparatus according to any one of claims 1-5, wherein a length of the side tube is 50 - 200 cm.

8. The apparatus according to any one of claims 1-7, wherein the inner diameter of the side-tube is less than an inner diameter of the infusion tubing.

9. The apparatus according to any one of claims 1-7, wherein the inner diameter of the side-tube is greater than or equal to an inner diameter of the infusion tubing.
